# EUROPEAN PATENT SPECIFICATION

(11) **EP 1 922 089 B1**
(45) Date of publication and mention of the grant of the patent: **24.12.2008**
(21) Application number: 06762656.4
(22) Date of filing: 17.07.2006
(51) Int. Cl.: A61L 15/28, A61L 15/22, A61L 15/46

(54) **BIOMATERIALS BASED ON CARBOXYMETHYLCELLULOSE SALIFIED WITH ZINC ASSOCIATED WITH HYALURONIC ACID DERIVATIVES**
BIOMATERIALIEN AUF DER BASIS VON CARBOXYMETHYLCELLULOSE IN ZINKSALZFORM IN ASSOZIATION MIT HYALURONSÄUREDERIVATEN
BIOMATERIAUX A BASE DE CARBOXYMETHYLCELLULOSE SALIFIEE AU ZINC ASSOCIE A DES DERIVES D'ACIDE HYALURONIQUE

(30) Priority: 22.07.2005 IT MI20051415
(43) Date of publication of application: 21.05.2008
(73) Proprietor: FIDIA ADVANCED BIOPOLYMERS S.R.L., 35031 Abano Terme (Padova) (IT)
(72) Inventor: BELLINI, Davide, I-25020 Albignasego (IT); TERRASSAN,Massimo, I-35020 Albignasego (IT); PAVESIO, Alessandra, I-35141 Padova (IT)
(74) Representative: Minoja, Fabrizio
(86) International application number: PCT/EP2006/007019
(87) International publication number: WO 2007/009728

(56) References cited:
- EP-A- 1 245 239
- EP-B1- 0 618 817
- WO-A-02/18448
- WO-A-96/13282
- WO-A-99/04828
- WO-A-02/076518

## Description

The present invention describes new biomaterials in the form of non-woven felts, comprising carboxymethylcellulose salified with zinc associated with hyaluronic derivatives at varying percentages, for use in surgery to treat various kinds of wounds, in particular infected wounds, pressure sores, bums, and in all conditions requiring the association of a wound healing and protective action with an antibacterial and/or antifungal action. The present invention also concerns the process for preparing the biomaterials themselves.

### BACKGROUND OF THE INVENTION

The treatment of wounds of various degrees of severity, characterised by infection or a high risk of infection, be they skin abrasions, bums or ulcers of various kinds (diabetic ulcers, pressure sores, venous or arterial ulcers), is performed by applying a series of medical devices intended to protect the lesion, favour wound healing, prevent necrosis, absorb any exudate that may form, and in particular release substances with antimicrobial/antibacterial/antifungal properties.

The devices currently on the market substantially contain silver metal as an antibacterial agent and, on the basis of their characteristics, can be subdivided into:
Hydrocolloids (adhesive and non-adhesive foams): for example, polyurethane foams associated with hydrocolloid substances (gelatine, pectin) such as Contreet^{®} by COLOPLAST.
Hypoallergenic tablets: such as Katomed^{®} with micronised silver metal buffer by DEVERGE' M.& M.
Polyethylene mesh: such as Acticoat^{®} (Smith & Nephew), multilayer, composed of an absorbent core between two non-adherent polyethylene meshes coated with nanocrystalline silver.
Dusting powders and saline solutions: such as Katoxyn^{®} (powder based on micronised, metallic silver) and Vulnopur^{®} (saline solution containing silver), both by DEVERGE' M.& M.
Hydrofibre dressings: such as Aquacel Ag, dressings made of Hydrofiber^{®} (cellulose carboxymethyl fibre) and ionic silver.

The devices to be used are chosen according to the requirements to be met and depending both on the type of wound and the quantity of silver to be released into it, that is, the level of infection or risk of infection.

Indeed, some conditions require the immediate application of a considerable amount of silver (severely infected lesions), others require slow but constant release of the metal (partial or full thickness pressure sores).

The use of silver-based dressings as described above is somewhat limited in that silver often gives rise to sensitisation phenomena, (see. Chronic exposure to Silver or Silver salts, Patty's Industrial Hygiene and Toxicology, Vol. 2°, G.D. Clayton, F.E. Clayton, Eds. Wiley-Interscience, New York, 3rd ed.,1981, pp 1881 - 1894), especially in cases requiring strong doses of metal to keep bacterial infections at bay, particularly in the case of bacterial and/or fungal strains such as S. *Aureus*; *E. coli, C. albicans; A. niger* and *P. aeruginosa.*

Non-woven felts based on fibres of hyaluronic acid derivatives, possibly in association with cellulose derivatives, have already been described as biomaterials, particularly for the purpose of forming clots. See for example EP 618817 which does not however describe biomaterials containing metal ions with antibacterial activity.

### DESCRIPTION OF THE INVENTION

The limitations and drawbacks of the known technique have been overcome by the biomaterials that are the subject of the present invention, obtainable by associating hyaluronic acid derivatives with carboxymethylcellulose (CMC) salified with zinc, prepared by a process providing devices characterised by bioadhesiveness, elasticity, absorbent properties, biodegradability/bioresorbability and broad spectrum antibacterial activity.

The devices of the present invention remain *in situ* long enough to allow the tissues on which they are applied to heal and, by absorbing the exudate from the wound without releasing it, they prevent bacterial contamination thanks to the presence of zinc in their fibres. Moreover, the dressings are easy to handle, can be adapted to fit wounds of any shape and size, and they are flexible, and therefore comfortable to wear.

According to the present invention, CMC salified with zinc is successfully associated with hyaluronic acid derivatives by a process that provides medical devices characterised by antimicrobial activity and suitable for the treatment of wounds of various origin.

It has long been known that zinc has antibacterial activity, and there have been numerous publications on the subject, such as:
- Yamamoto et. al. "Effect of lattice constant of zinc oxide on antibacterial characteristics" J Mater Sci Mater Med. 2004, 15, 847-851
- Cho YH et. al. "Antibacterial effect of intraprostatic zinc injection in a rat model of chronic bacterial prostatitis" Int J Antimicrob Agents. 2002, 19, 576-582
- G.M. Hill et. al. "Effect of pharmacological concentrations of zinc oxide with or without the inclusion of an antibacterial agent on nursery pig performance " J Anim. Sci 2001, 79, 934-941
while other publications describe its ability to enhance wound healing:
- Kerry A. et. al. "Zinc-containing wound dressings encourage autolytic debridement of dermal burns " WOUNDS 1998, 10, 54-58
- Joe A. Vinson et. Al. "Age related differences in topical zinc absorption from different formulations: Implications for health and disease" J Geriatr Dermatol 1997, 5,276-280.
Moreover, EP 526541 discloses that zinc bound to acid resins is useful for the treatment of oral cavity diseases. Said resins are reported to have very low allergenic properties.

The toxicology of zinc has been widely documented (DRAFT TOXICOLOGICAL PROFILE FOR ZINC, U.S. DEPARTMENT OF HEALTH AND HUMAN SERVICES, Public Health Service Agency for Toxic Substances and Disease Registry September 2003).

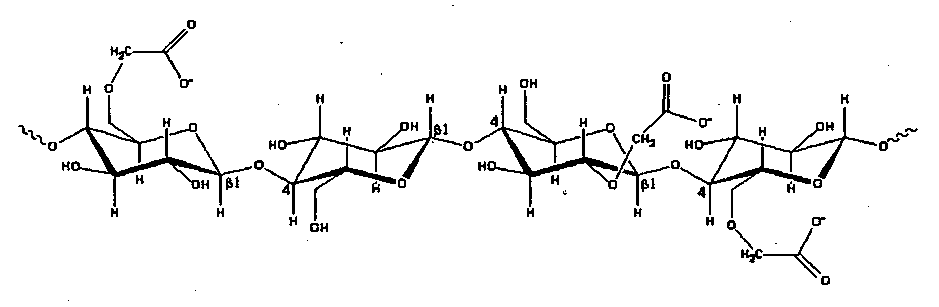

### Structure of Carboxymethyl cellulose

Carboxymethyl cellulose is a semisynthetic hydrophilic cellulose derivative (sodium salt of the polycarboxymethyl ether of cellulose) with a high degree of viscosity and a MW that varies between 21,000 and 500,000, which looks like a granular or fibrous powder, white, yellowish or greyish, slightly hygroscopic, odourless and tasteless.

The polymers contain substituted units of anhydrous glucose with the following general formula:C₆H₇O₂(OR1)(OR2)(OR3) where R1, R2, R3 may be:-H,- CH₂COONa,- CH₂COOH.

It is widely used as an agent in suspensions and emulsions, as an excipient for tablets and as an agent to increase viscosity in pharmaceutical preparations. Besides being present in numerous injectable steroid preparations, it is administered by the oral route as a cathartic, and by the oral or rectal routes as a component in barium meal used as a contrast medium in X-rays. The presence of circulating specific IgE for carboxymethylcellulose has been demonstrated, and patients who experienced reactions immediately after taking steroids containing this excipient tested positive in the skin prick test.

Hyaluronic acid (HA) has long been known. It is a heteropolysaccharide consisting of alternating residues of D-glucuronic acid and N-acetyl-D-glucosamine. It has a linear chain and a molecular weight ranging between 50,000 and 13 x 10⁶ Da, according to the source it was extracted from and/or the method used for its preparation. It is present in nature in the pericellular gels, in the fundamental substance of the connective tissue in vertebrate organisms (of which it is one of the chief components), in the synovial fluid of joints, the vitreous humor and umbilical cord.

HA is therefore fundamentally important to the biological organism, especially as a mechanical support for the cells of many tissues such as the skin, tendons, muscle and cartilage.

Through its membrane receptor, CD44, hyaluronic acid modulates many different processes relative to the physiology and biology of cells, such as cell proliferation, migration, differentiation and angiogenesis, besides its other functions, such as tissue hydration and joint lubrication. Moreover, it has been demonstrated that HA plays a fundamental role in the tissue repair process both from a structural point of view (in the organisation of the extracellular matrix and in regulating its hydration) and in stimulating a wide series of processes in which it intervenes directly and indirectly (clot formation, phagocyte activity, fibroblast proliferation, neovascularisation, reepithelialisation, etc.) (Weigel P. et al., J Theoretical Biol, 1986:219-234; Abatangelo G. et al., J Surg Res, 1983, 35:410-416; Goa K. et al., Drugs, 1994, 47:536-566).

These widely acknowledged properties have long been exploited for the preparation of dressings for wounds, ulcers and skin lesions of various origin.

Hyaluronic acid has been chemically modified in various ways, giving polymers that maintain the biological/pharmacological characteristics of the starting polymer, but are easier to process and give a better mechanical performance. Particularly suitable for the purposes of the present invention are the hyaluronic acid derivatives obtained by:
esterification with alcohols of the aliphatic, araliphatic, cycloaliphatic, aromatic, cyclic and heterocyclic series, with a percentage of esterification which may vary between 0.1 and 100%, preferably between 50 and 100%, according to the type and length of the alcohol used, while the remaining percentage of non-esterified HA may be salified with organic and/or inorganic bases (HYAFF^{®}- EP 216453 B1). The excellent absorbent properties of these materials have also been recognised (EP 999859 B1);
percarboxylation, obtained by oxidating the primary hydroxyl of the N-acetyl-glucosamine fraction with a degree of percarboxylation of between 0 and 100% and preferably between 25 and 75% (HYOXX^{™} - patent application No. EP 1339753).

The hyaluronic acid used in the present invention may be obtained from any source, for example by extraction from rooster combs (EP 13 8572 B1), or by fermentation (EP 716688 B1), or by technological means, and its molecular weight may vary within a range of 400 to 3x10⁶Da.

The derivatives described here are associated with CMC zinc salt at suitable percentages and, by means of a wet extrusion process, fibres are obtained therefrom which are used to make the new biocompatible and biodegradable, highly absorbent, elastic, flexible, bioadhesive medical devices, which can be adapted to fit wounds of any shapes and sizes and of various kinds, and have antibacterial and/or antifungal activity.

### DETAILED DESCRIPTION OF THE INVENTION

The present invention relates to new biomaterials and to the process for their preparation, in the form of non-woven felts, suitable for the preparation of medical devices to be used singly or in association with others, in the treatment of wounds of various kinds (for example, skin abrasions, deep excoriations, cuts) and burns that are infected or at risk of becoming infected. The biomaterials of the present invention are completely biocompatible and biodegradable (they do not therefore need to be removed from the application site), bioadhesive, elastic, flexible, adaptable to fit the wound, highly absorbent and able to exercise high antibacterial and/or antifungal activity. These characteristics are due to the polymers that constitute the new biomaterials, that is, CMC salified with zinc and a hyaluronic acid derivative, mixed together at given percentages and processed by wet extrusion as described below.

As said before, CMC is able to form hydrogels when cellulose is carboxylated to a degree of between 15 and 40%; these are the ideal conditions in which to obtain compounds with excellent properties for the absorption of physiological aqueous solutions.

It is well known that hyaluronic acid has been chemically modified in various ways to obtain polymers that maintain the biological/pharmacological properties of the starting polymer, but with better mechanical properties (for example, better resistance and easier processing and handling) and with adjustable biodegradability.

Particularly preferred derivatives of the present invention, in that they are easy to hydrate, are those obtained by:
- Esterification with alcohols of various kinds, particularly with benzyl alcohol (HYAFF^{®-} 11), esterified to a degree of between 0.1 and 100%, preferably between 50 and 100%;
- percarboxylation (HYOXX^{™}) of the N-acetyl-glucosamine fraction with a degree of percarboxylation of between 0 and 100% and preferably between 25 and 75%.

The hydratability of the hyaluronic acid fraction is fundamental for maintaining a moist environment favouring wound healing and supporting the action of the CMC zinc salt in absorbing exudate, so as to prevent infections, necrosis of the new tissue and abnormal scarring. Hyaluronic acid exercises its biological/pharmacological action by intervening on various fronts. It is in fact active in organising the extracellular matrix and in regulating its hydration, and it stimulates a wide series of processes in which it intervenes directly or indirectly (clot formation, phagocyte activity, fibroblast proliferation, neovascularisation, re-epithelialisation). Its prolonged presence *in situ* due to the slow biodegradability of CMC enhances these combined effects.

If desired, further polymers such as alginic acid or salts thereof, gellan and collagen may be added to CMC and hyaluronic acid derivatives.

The process for the preparation of the biomaterial according to the invention allows an efficient processing of the polymers making them easily adapted to industrial requirements. It also results in medical devices with advantageous features in comparison to the known products currently available.

The process of the invention comprises the wet-extrusion of a mixture of polymers in suitable conditions. In particular, the process comprises two separate steps:
1 - preparation of the solution of the polymers in an apolar aprotic solvent (e.g. dimethylsulphoxide - DMSO).
   The solution is prepared by mixing ammonium derivatives of CMC, such as the tetrabutylammonium salt or benzalkonium salt, with hyaluronic acid derivatives in a percentage of 95 and 5% respectively, preferably 90 and 10% and more preferably 80 and 20%. CMC used is a commercially available such as Walocel^{®} CRT 1000 (Bayer), while the hyaluronic acid derivative is an ester, preferably a benzyl ester, esterified to varying degrees, for example 75% (HYAFF^{®-} 11p75) or 100% (HYAFF^{®} 11). A further polymer may be added to said mixture, e.g. alginic acid or gellan in salified forms as well (tetrabutylammonium or benzalkonium), in percentages ranging from 5 to 50% by weight of the CMC/Hyaff® mixture. Gellan is commercially available from CP Kelco US ( Kelcogel CG-LA Gellan Gum) whereas alginic acid, particularly sodium alginate, is manufactured by PRONOVA. The products are then dissolved at concentrations of between 100 and 140 mg/ml in an aprotic apolar solvent (DMSO).
2 - extrusion

The solution is placed in a tank and fed through a metering pump to a wet extrusion spinneret with 3000 holes each measuring 65 µ in diameter.

Extrusion takes place in a coagulation bath containing zinc chloride or zinc bromide in a 5-10% alcohol solution (absolute ethanol) .The mass of threads coming out from the spinneret is transported by rollers into two successive rinsing baths containing absolute ethanol alone.

At the end of the rinsing stage, the mass of threads is dried in a hot air stream.

The fibres thus obtained are carded to obtain a non-woven felt.

The fibres obtained by said process have a diameter varying between 10 and 60 µ, while the non-woven felt may weigh between 20 and 500 g/m² and have a width of between 0.2 and 5 mm.

The biomaterial can be sterilised in the packaging stage by the normal methods (e.g. γ ray).

Detailed examples for the preparation of fibres of the invention are reported below.

### Example 1. Preparation of a non-woven felt composed of a mixture of CMC zinc salt (90%) and HYAFF^{®} 11 p75 (10%)

9 g of CMC tetrabutylammonium salt obtained by ion exchange on strong sulphonic resin Dowex M 15 loaded with tetrabutylammonium hydroxide starting from CMC Walocel^{®} CRT 1000 was mixed with 1 g of HYAFF^{®} 11 p75 in powder form and solubilised with 100 ml of DMSO.

Once solubilisation was complete, the solution was placed in a tank and fed through a metering pump to a wet extrusion spinneret with 3000 holes each measuring 65 µ. Extrusion occurred in a coagulation bath containing zinc chloride in an alcohol solution of 5% absolute ethanol.

The mass of threads coming out from the spinneret was transported by rollers into two successive rinsing baths containing absolute ethanol, and then dried in a current of hot air.

The fibres thus obtained were carded to obtain a non-woven felt.

The final non-woven felt was 2 mm thick and was cut into pieces of 10X10 cm and sterilised by γ ray.

### Example 2. Preparation of a non-woven felt composed of a mixture of CMC zinc salt (95%) and HYAFF^{®} 11 p75 (5%)

19 g of CMC benzalkonium salt, obtained by ion exchange with benzalkonium chloride starting from CMC Walocel^{®} CRT 1000, was mixed with 1 g of HYAFF^{®} 11 p75 in powder form and solubilised with 160 ml of DMSO.

Once solubilisation was complete, the solution was placed in a tank and fed through a metering pump to a wet extrusion spinneret with 3000 holes, each measuring 65 µ. Extrusion occurred in a coagulation bath containing zinc bromide in an alcohol solution of 7% absolute ethanol.

The mass of threads coming out from the spinneret was transported by rollers into two successive rinsing baths containing absolute ethanol, and then dried in a current of hot air.

The fibres thus obtained were carded to obtain a non-woven felt.

The final non-woven felt was 1 mm thick and was cut into pieces of 5X5 cm and sterilised by γ ray.

### Example 3. Preparation of a non-woven felt composed of a mixture of CMC zinc salt (95%) and HYOXX^{®}(5%)

9.5 g of CMC benzalconium salt, obtained by ion exchange with benzalconium chloride starting from CMC Walocel^{®} CRT 1000, were mixed with 0.5 g of HYOXX^{®} in powder form, and solubilised with 100 ml of DMSO.

Once solubilisation was complete, the solution was placed in a tank and fed through a metering pump to a wet extrusion spinneret with 3000 holes, each measuring 65 µ. Extrusion occured in a coagulation bath containing zinc chloride in an alcohol solution of 5% absolute ethanol.

The mass of threads coming out from the spinneret was transported by rollers into two successive rinsing baths containing absolute ethanol, and then dried in a hot air stream.

The fibres thus obtained were carded to obtain a non-woven felt.

The final non-woven felt was 1 mm thick and was cut into pieces of 5X5 cm and sterilised by γ ray.

### Example 4. Preparation of a non-woven felt composed of a mixture of CMC zinc salt (90%) and HYAFF^{®} 11 p75 10% and alginic acid (40% on the CMC/HYAFF^{®} mixture

9 g of CMC tetrabutylammonium salt obtained by ion exchange on strong sulphonic resin Dowex M 15 loaded with tetrabutylammonium hydroxide starting from CMC Walocel^{®} CRT 1000 was mixed with 1 g of HYAFF^{®} 11 p75 in powder form and solubilised with 100 ml of DMSO. 4 g of alginic acid benzalkonium salt, obtained by ion exchange with benzalkonium chloride, was solubilised separately in 40 ml of DMSO and added to the CMC/HYAFF^{®} mixture, thereby obtaining a mixture added with alginic acid in a 40% amount on the CMC/HYAFF^{®} mixture weigh.

The resulting mass was placed in a tank and fed through a metering pump to a wet extrusion spinneret with 3000 holes each measuring 65 µ. Extrusion occurred in a coagulation bath containing zinc chloride in an alcohol solution of 5% absolute ethanol.

### Example 5. Preparation of a non-woven felt composed of a mixture of CMC zinc salt (80%) and HYAFF^{®} 11 p75 (20%) and gellan (25% on the CMC/HYAFF^{®} mixture

8 g of CMC tetrabutylammonium salt obtained by ion exchange on strong sulphonic resin Dowex M 15 loaded with tetrabutylammonium hydroxide starting from CMC Walocel^{®} CRT 1000 was mixed with 2 g of HYAFF^{®} 11 in powder form and solubilised with 100 ml of DMSO. 2.5 g of gellan, obtained by ion exchange on strong sulphonic resin Dowex M 15 loaded with tetrabutylammonium hydroxide starting from Kelcogel CG-LA, in 25 ml of DMSO was solubilised separately in 25 ml of DMSO and added to the CMC/HYAFF^{®} mixture, thereby obtaining a mixture added with gellan in a 25% amount on the CMC/HYAFF^{®} mixture weigh.

The resulting mass was placed in a tank and fed through a metering pump to a wet extrusion spinneret with 3000 holes each measuring 65 µ. Extrusion occurred in a coagulation bath containing zinc chloride in an alcohol solution of 5% absolute ethanol.

Each of the non-woven felts thus prepared was subjected to specific tests to assess both the quantity of zinc contained and released by the CMC fibres and to demonstrate how effective the zinc is in inhibiting the growth of some bacterial and fungal species.

The tests are described below.

### Evaluation of the zinc ion content in non-woven felt of CMC zinc salt with HYAFF^{®}11p75 in a 95/5 mixture, after treatment in artificial plasma at 37°C.

The non-woven felt obtained as described in Example 2 was tested for the release kinetics of the zinc ion present in the fibre, by treatment with artificial plasma at a temperature of 37°C and at set intervals of 2, 4, 6, 24, 48 and 72 hours.

100 mg of non-woven felt were placed in 6 10-ml sterile glass vials and then treated with 5 ml of artificial plasma. All the vials were kept at a temperature of 37°C for the set time intervals. After the period of incubation, each sample was filtered through a 0.2 µ filter, and the solution was freeze-dried. For time 0, artificial plasma alone was filtered through a 0.2 µ filter and freeze-dried.

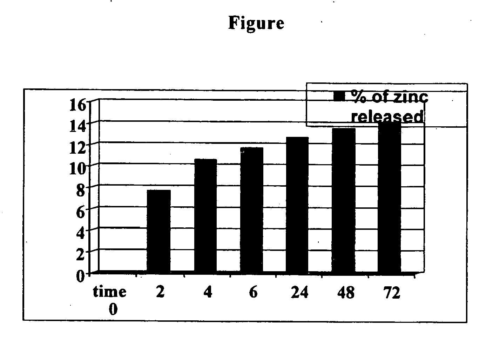

The quantity of zinc released during the experiment is shown in Figure 1.

Zinc is released from the non-woven felt consistently over the first 2 hours in contact with the artificial plasma (7.6% vs. a total of 14% which is the percent of zinc ions present in the fibres), and then progressively over the next 48-72 hours until all zinc has been released.

### Analysis by agar diffusion of the inhibitory activity of a non-woven felt of CMC zinc salt with HYAFF^{®}11p75 in a 90/10 mixture.

The non-woven felt obtained according to Example 1 was tested for its antibacterial activity against strains of *P. aeruginosa; E. coli; S. aureus; C. albicans, A. niger.*

As negative control, Walocel^{®} CRT 1000 as starting CMC was used.

The procedure was repeated, in aseptic conditions, for each microbial strain.

Two Petri dishes were prepared, each with a layer of 15 ml of solidified, specific, incomplete agar medium, added with 5 ml of specific nutrient medium melted at 45°C, previously inoculated with the test micro-organism to obtain a concentration in the medium of about 10⁴ UFC/ml. The medium was left to solidify.

Three wells, 10 mm in diameter, were made in each Petri dish.

One dish was used for the sample and the other for the negative control.

The test sample and the negative control were prepared as follows: 0.6 g was dissolved in 9 ml of sterile distilled water to give a mixture of gelatinous consistency. The wells made in the Petri dishes were filled to the brim with aliquots of this mixture (about 100 µL).

In parallel to the test procedure described above, each microbial strain was tested for fertility of the medium by preparing a Petri dish with a layer of less than 15 ml of specific, incomplete agar medium and a layer of over 5 ml of specific nutrient medium previously inoculated with the test micro-organism, so as to obtain a concentration of about 10⁴ UFC/ml in the medium.

The dishes set up in this way were incubated at 30°C ± 1°C for 48-72 hours.

At the end of the incubation period, the dishes were inspected visually for the presence of any inhibiting haloes around the wells. Moreover, microbial growth was assessed in the dishes prepared to control fertility of the medium.

An evident inhibiting halo was seen in the dishes containing samples of *S. aureus, E. coli, C. albicans* and *A. niger,* while a somewhat more limited halo was seen in the dish inoculated with *P. aeruginosa.*

No inhibition halo was detected in any of the negative control dishes.

Microbial growth was observed in all the dishes testing fertility of the medium.

In conclusion, in the test conditions described above, the test product proved to have excellent bactericidal activity towards the strains *S*. *aureus* and *E. coli,* more moderate bacteriostatic activity towards *P. aeruginosa* and fungicide activity towards *A*. *niger and C. albicans*.

## Claims

1. Biomaterials in the form of non-woven felt constituted by an association of hyaluronic acid derivatives with carboxy methylcellulose (CMC) salified with zinc.

2. Biomaterials according to claim 1 wherein the hyaluronic acid derivatives are esters with alcohols.

3. Biomaterials according to claim 2 wherein the hyaluronic acid derivatives are benzyl esters.

4. Biomaterials according to claim 2 or 3 with a percentage of esterification of between 0.1 and 100%.

5. Biomaterials according to claim 4 with a percentage of esterification of between 50 and 100%.

6. Biomaterials according to claim 1 wherein the hyaluronic acid derivatives are percarboxylated derivatives of the N-acetyl-glucosamine fraction.

7. Biomaterials according to claim 6 with a degree of percarboxylation of between 25 and 75%.

8. Biomaterials according to any one of claims 1 to 7 wherein the carboxy methylcellulose has a degree of carboxylation of between 15 and 40%.

9. Biomaterials according to any one of claims 1 to 8 wherein the percentage in weight of carboxy methylcellulose is between 95 and 5% that of the hyaluronic acid derivative.

10. Biomaterials according to claim 9, wherein the percentage in weight of the carboxy methylcellulose is between 90 and 10% that of the hyaluronic acid derivative.

11. Biomaterials according to claim 10, wherein the percentage in weight of the carboxy methylcellulose is between 80 and 20% that of the hyaluronic acid derivative.

12. Biomaterials according to any one of claims 1-11, further comprising a polymer selected from alginic acid or salts thereof, gellan and collagen .

13. Process for the preparation of biomaterials of claims 1-12, which includes:
a) preparing a solution of ammonium salts, of carboxy methylcellulose and of the hyaluronic acid derivative and optionally of the further polymer of claim 12 in an aprotic apolar solvent;
b) wet-extrusion in a spinneret in a coagulation batch containing zinc chloride or zinc bromide in an alcohol solution of 5 to 10% ethanol;
c) rinsing the threads obtained by extrusion in ethanol;
d) drying in a hot air stream;
e) carding the dried threads.

14. Process according to claim 13 in which the carboxy methylcellulose salts are tetrabutylammonium or benzalkonium salts.

15. Process according to claim 13 or 14 in which the solvent is dimethylsulphoxide.

16. Fibres obtainable by the process of claims 13 to 15 which have a diameter varying between 10 and 60 µ.

17. Use of the biomaterials of claims 1-12 for the preparation of tools or devices for the treatment of wounds of various origin and bums.

## Patentansprüche

1. Biomaterialien in der Form von nicht gewebtem Filz, zusammengesetzt aus einer Assoziation von Hyaluronsäure-Derivaten mit mit Zink salifizierter Carboxymethylcellulose (CMC).

2. Biomaterialien gemäß Anspruch 1, wobei die Hyaluronsäure-Derivate Ester mit Alkoholen sind.

3. Biomaterialien gemäß Anspruch 2, wobei die Hyaluronsäure-Derivate Benzylester sind.

4. Biomaterialien gemäß Anspruch 2 oder 3 mit einem Veresterungsanteil von 0,1 bis 100%.

5. Biomaterialien gemäß Anspruch 4 mit einem Veresterungsanteil von 50 bis 100%.

6. Biomaterialien gemäß Anspruch 1, wobei die Hyaluronsäure-Derivate percarboxylierte Derivate der N-Acetylglucosamin-Fraktion sind.

7. Biomaterialien gemäß Anspruch 6 mit einem Percarboxylierungsgrad von 25 bis 75%.

8. Biomaterialien gemäß einem der Ansprüche 1 bis 7, wobei die Carboxymethylcellulose einen Carboxylierungsgrad von 15 bis 40% aufweist.

9. Biomaterialien gemäß einem der Ansprüche 1 bis 8, wobei der Gewichtsanteil von Carboxymethylcellulose 95 bis 5% von dem des Hyaluronsäure-Derivats beträgt.

10. Biomaterialien gemäß Anspruch 9, wobei der Gewichtsanteil der Carboxymethylcellulose 90 bis 10% von dem des Hyaluronsäure-Derivats beträgt.

11. Biomaterialien gemäß Anspruch 10, wobei der Gewichtsanteil der Carboxymethylcellulose 80 bis 20% von dem des Hyaluronsäure-Derivats beträgt.

12. Biomaterialien gemäß einem der Ansprüche 1-11, ferner umfassend ein Polymer, ausgewählt aus Alginsäure oder Salzen davon, Gellan und Kollagen.

13. Verfahren zur Herstellung von Biomaterialien nach Ansprüchen 1-12, das beinhaltet:
a) Herstellen einer Lösung von Carboxymethylcelluloseammoniumsalzen und dem Hyaluronsäure-Derivat und gegebenenfalls dem weiteren Polymer nach Anspruch 12 in einem aprotischen apolaren Lösungsmittel;
b) Nassextrudieren in einer Spinndüse in einem Koagulationsbad, der Zinkchlorid oder Zinkbromid in einer Alkohollösung von 5 bis 10% Ethanol enthält;
c) Spülen der durch Extrudieren in Ethanol erhaltenen Fäden;
d) Trocknen in einem heißen Luftstrom;
e) Kardieren der getrockneten Fäden.

14. Verfahren gemäß Anspruch 13, bei dem die Carboxymethylcellulosesalze Tetrabutylammonium- oder Benzalkoniumsalze sind.

15. Verfahren gemäß Anspruch 13 oder 14, bei dem das Lösungsmittel Dimethylsulfoxid ist.

16. Fäden, erhältlich durch das Verfahren nach Ansprüchen 13 bis 15, die einen Durchmesser aufweisen, der von 10 bis 60 µ variiert.

17. Verwendung der Biomaterialien nach Ansprüchen 1-12 zur Herstellung von Instrumenten oder Vorrichtungen zur Behandlung von Wunden verschiedenen Ursprungs und Verbrennungen.

## Revendications

1. Biomatériaux sous forme d'un feutre non tissé constitués d'une association de dérivés d'acide hyaluronique avec de la carboxyméthyl cellulose (CMC) salifiée avec du zinc.

2. Biomatériaux selon la revendication 1, dans lesquels les dérivés d'acide hyaluronique sont des esters avec des alcools.

3. Biomatériaux selon la revendication 2, dans lesquels les dérivés d'acide hyaluronique sont des esters de benzyl.

4. Biomatériaux selon la revendication 2 ou 3 avec un pourcentage d'estérification compris entre 0.1 et 100%.

5. Biomatériaux selon la revendication 4 avec un pourcentage d'estérification compris entre 50 et 100%.

6. Biomatériaux selon la revendication 1, dans lesquels les dérivés d'acide hyaluronique sont des dérivés percarboxylés de la fraction N-acétyl-glucosamine.

7. Biomatériaux selon la revendication 6 avec un degré de percarboxylation compris entre 25 et 75%.

8. Biomatériaux selon l'une quelconque des revendications 1 à 7, dans lesquels la carboxyméthyl cellulose a un degré de carboxylation compris entre 15 et 40%.

9. Biomatériaux selon l'une quelconque des revendications 1 à 8, dans lesquels le pourcentage en poids de carboxyméthyl cellulose est compris entre 95% et 5% par rapport aux dérivés d'acide hyaluronique.

10. Biomatériaux selon la revendication 9, dans lesquels le pourcentage en poids de carboxyméthyl cellulose est compris entre 90% et 10% par rapport aux dérivés d'acide hyaluronique.

11. Biomatériaux selon la revendication 10, dans lesquels le pourcentage en poids de carboxyméthyl cellulose est compris entre 80% et 20% par rapport aux dérivés d'acide hyaluronique.

12. Biomatériaux selon l'une quelconque des revendications 1 à 11, comprenant en outre un polymère choisi parmi l'acide alginique ou des sels de celui-ci, la gellane et le collagène.

13. Procédé pour la préparation de biomatériaux selon les revendications 1 à 12, incluant :
a) la préparation d'une solution de sels d'ammonium de carboxyméthyl cellulose et du dérivé d'acide hyaluronique, et éventuellement du polymère supplémentaire de la revendication 12, dans un solvant apolaire aprotique.
b) l'extrusion humide, dans une filière, d'un lot de coagulation contenant du chlorure de zinc ou du bromure de zinc dans une solution d'alcool de 5 à 10% d'éthanol ;
c) le rinçage des fils obtenus par extrusion dans l'éthanol ;
d) le séchage dans un courant d'air chaud ;
e) le cardage des fils séchés.

14. Procédé selon la revendication 13, dans lequel les sels de carboxyméthyl cellulose sont des sels de tétrabutylammonium ou de benzalkonium.

15. Procédé selon la revendication 13 ou 14, dans lequel le solvant est le diméthylsulphoxide.

16. Fibres obtenus à partir du procédé des revendications 13 à 15 ayant un diamètre variant entre 10 et 60 µ.

17. Utilisation des biomatériaux selon les revendications 1 à 12 pour la préparation d'outils ou de dispositifs pour le traitement de plaies d'origine variée ou de brûlures.
